# EUROPEAN PATENT SPECIFICATION

(11) **EP 4 025 192 B1**
(45) Date of publication and mention of the grant of the patent: **03.12.2025**
(21) Application number: 20764666.2
(22) Date of filing: 02.09.2020
(51) Int. Cl.: A61K 38/26

(54) **PROCESS FOR PRODUCING A TABLET COMPRISING GLP-1 PEPTIDES**
VERFAHREN ZUR HERSTELLUNG EINER TABLETTE MIT GLP-1-PEPTIDEN
PROCÉDÉ DE PRODUCTION D'UN COMPRIMÉ COMPRENANT DES PEPTIDES GLP-1

(30) Priority: 02.09.2019 EP 19194868
(43) Date of publication of application: 13.07.2022
(73) Proprietor: Novo Nordisk A/S, 2880 Bagsværd (DK)
(72) Inventor: INGVARSSON, Pall, Thor, 2880 Bagsværd (DK)
(86) International application number: PCT/EP2020/074426
(87) International publication number: WO 2021/043803

(56) References cited:
- WO-A1-2012/080471
- WO-A1-2012/140117
- WO-A1-2013/189988
- WO-A1-2015/155151
- WO-A2-2010/072621

## Description

The present invention relates to a process for producing a tablet comprising a GLP-1 peptide, wherein the GLP-1 peptide in the tablet is obtained by spray-drying of a feed solution comprising a GLP-1 peptide and a feed solution solvent, wherein the pH of the feed solution is in the range of about 5 to about 10 or wherein the pH of the feed solution is higher than the pl of the GLP-1 peptide. More specifically, the invention pertains to a process for producing a tablet comprising a GLP-1 peptide, wherein the tablet obtained has an increased dissolution rate, a tablet obtained by said process and its use in medicine.

### BACKGROUND

A convenient and frequent way of administering drugs is via the oral route. Generally, dosage forms comprise an active pharmaceutical ingredient (API) and excipients, which help to ensure the drug reach its site of action, prevent unwanted decomposition and in some cases controlling release. To exert a therapeutic effect the drug must dissolve in the aqueous environment of the gastrointestinal tract.

The effectiveness of an oral dosage form often depends upon the intrinsic ability of a drug to dissolve in the gastrointestinal tract prior to being absorbed into the circulation. Drug solubility and dissolution are important parameters affecting the bioavailability. Dissolution refers to a dynamic process-involving the kinetics by which a material dissolves into a given media (e.g., solvent). Viewed from the perspective of the resulting solution, dissolution can be characterised by the time-rate-of-change in concentration of the sample material in the solution over a dissolution period. In contrast, "solubility" generally refers to an equilibrium condition (e.g., a thermodynamic value) and particularly refers to how much of a sample material will dissolve in a given medium under conditions in which thermodynamic equilibrium is achieved. In general, materials that have a high solubility will generally demonstrate faster dissolution than materials of lower solubility. However, dissolution characteristics do not directly and specifically correlate to solubility, and valuable information can be obtained by looking at dissolution profiles (e.g., in addition to overall solubility data).

Human GLP-1 and GLP-1 peptides generally have a low bioavailability and their exposure following oral administration is low. In order to increase bioavailability certain absorption enhancers have been used (Am J Clin Nutr; Oct 2010; 92; 810-817; WO2010/020978; WO2012/080471; WO2013/189988; WO2013/139695). However, such orally formulated GLP-1 peptides still need to be taken some time in advance of eating, drinking or taking other oral medications in order to ensure that the active ingredient is taken up e.g. oral GLP-1 peptide semaglutide is to be taken 30 min. in advance of a meal to ensure maximum effect. Therefore, there is still a need for improving absorption and bioavailability of such tablets comprising GLP-1 peptides.

### SUMMARY

In some embodiments, the invention relates to a process of producing a tablet comprising a GLP-1 peptide, said method comprising the steps of a) spray-drying a feed solution comprising the GLP-1 peptide and a feed solution solvent, wherein the pH of the feed solution is in the range of about 5 to about 10 or wherein the pH of the feed solution is higher than the pl of the GLP-1 peptide; and b) compressing the obtained powder of GLP-1 peptide and optionally at least one pharmaceutically acceptable excipient into a tablet.

In some embodiments, the invention relates to a tablet comprising a GLP-1 peptide, wherein the GLP-1 peptide is obtained by the process defined herein.

In some embodiments, the invention relates to an oral dosage form such as a tablet obtained by the process described herein for use in medicine, such as for the treatment of diabetes or obesity. In some embodiments, the invention relates to a method for treatment of diabetes or obesity comprising administering the oral dosage form such as the tablet obtained by the process as defined herein to a subject.

### BRIEF DESCRIPTION OF DRAWINGS

Fig. 1 shows the results of intrinsic dissolution test of a tablet comprising semaglutide obtained from spray-drying at given pH values.
Fig. 2 shows the results of intrinsic dissolution test of a tablet comprising compound A obtained from spray-drying at given pH values. * comprises NaCl; ^{#} had larger particles; ^{##} had smaller particles.

### DESCRIPTION

Many pharmaceuticals and surgical-aid products contain dry drug substances, which are usually dried from their liquid phase to their dry phase by freeze drying.

Spray-drying involves the atomisation of a liquid feed into very small droplets within a hot drying gas leading to flash drying of the droplets into solid particles. The particles are then separated from the drying gas, using e.g. a cyclone and/or a filter bag, as a final spray-dried product. The feed can be a solution, a suspension or an emulsion and the resulting product can be classified as a powder. Such a powder may be defined as comprising solid discrete particles; the powder may further be defined by a given size, shape, and morphology. A granule is a subclass of powder and may be defined as a particle consisting of smaller discrete first particles gathered together into a new second particle or larger aggregate. In a single continuous step, spray-drying therefore converts a liquid feed into a powder. Once the liquid has been spray-dried the resultant powder is compatible with many ingredients which would have broken down the matrix during spray-drying.

An advantage of spray-drying is that it allows for processability at lower temperatures, if needed, and also allows for other modifications to the process in order to further improve powder properties. A spray-dried powder can also be formulated further, if needed. In particular, the spray-dried powder may be formulated into a capsule, tablet or any other solid dosage form. Properties such as level of moisture or residual solvent in a spray-dried powder, particle morphology or size and powder density can be manipulated to a great extent to target levels. More specifically, the selection of suitable spray-drying conditions has a major impact on product properties, viz residual water content, T_{g}, particle size and morphology, and the extent of protein aggregation and/or activation. Important process variables to consider when optimising a spray-drying process are inlet and outlet temperature, feed rate and atomizing gas flow rate.

Other similar techniques to spray-drying include freeze-drying and vacuum drying. To freeze-dry a composition, it is cooled until it solidifies and placed under reduced pressure to cause the most volatile ingredients in the composition to sublime. The solid residue may form a single mass which requires milling to form a fine powder. A typical freeze-dried powder comprises porous irregular shaped particles and readily hydrates. The process for the production of the lyophilised drug substance is a batch process which usually lasts for several days and is limited by the capacity of the lyophiliser size. This puts the entire drying batch at risk for a significant amount of time until the process is completed. Furthermore, the lyophilised drug substance produced is in the form of a "cake" which needs further processing steps, including breaking, sieving and/or milling to obtain a powder. These steps may decrease the potency and mass yield of the dry product. The term "cake" or "solid cake" may refer to a porous and spongy structure like composition resulting from the lyophilisation process.

Advantageously, using a spray-drying process instead of lyophilisation provides a cost effective, continuous and high-capacity process, in which the dry product is immediately obtained in a form of dry powder and not as a "cake". Powders produced by freeze-drying over spray-drying are often about double the cost due to the extra complexity of the freeze-drying process (e.g. equipment to operate at reduced pressures). Furthermore spray-dried materials generally take the form of homogeneous powders, which are less hygroscopic than those obtained by freeze-drying.

Dissolution rate of an active ingredient in a tablet is of high importance in order for the active ingredient to be available for absorption and thereby being able to exert a maximum effect. Dissolution rate can be controlled by a variety of means e.g. by excipients resulting in a controlled release of the active ingredient. Other techniques utilise pH dependency, where active ingredient is withheld at certain pH values and released at other pH values. When an increased dissolution rate is obtained, the active ingredient is also absorbed faster in the body, which is beneficial for the patient if e.g. an oral dosage form such as a tablet needs to be taken in advance of a meal. By obtaining a faster dissolution rate, a faster absorption takes place and the time from taking the oral dosage form such as tablet to initiating the meal can be shortened improving usability and compliance for the patient.

The present inventors have surprisingly observed that when spray-drying a feed solution comprising a GLP-1 peptide and a feed solution solvent, wherein the feed solution has a pH higher than the pl of the GLP-1 peptide or wherein the pH is the range of about 5 to about 10, a tablet made with the resulting GLP-1 peptide powder has a higher dissolution rate when compared to a tablet made from the GLP-1 peptide spray-dried from a feed solution having a pH lower than pl of the GLP-1 peptide or a tablet made from the GLP-1 peptide spray-dried from a feed solution having a pH below about 5.

Accordingly, in some embodiments the invention relates to a process for producing a GLP-1 peptide such as semaglutide or compound A, wherein the GLP-1 peptide is obtained by spray-drying of a feed solution comprising said GLP-1 peptide and a feed solution solvent, wherein the pH of the feed solution has a pH higher than the pl of the GLP-1 peptide or wherein the pH of the feed solution has a pH in the range of about 5 to about 10, and the resulting GLP-1 peptide powder having an improved dissolution rate.

Accordingly, in some embodiments, the invention relates to a process for producing a tablet comprising a GLP-1 peptide, wherein the GLP-1 peptide is obtained by spray-drying of a feed solution comprising said GLP-1 peptide and a feed solution solvent, wherein the pH of the feed solution has a pH higher than the pl of the GLP-1 peptide wherein the pH of the feed solution has a pH in the range of about 5 to about 10, the resulting tablet having an improved dissolution rate.

### Process for producing a tablet

In some embodiments, the invention relates to a process for producing a tablet comprising a GLP-1 peptide, said process comprising the steps of a) spray-drying a feed solution comprising the GLP-1 peptide and a feed solution solvent, wherein the pH of the feed solution is higher than the pl of the GLP-1 peptide or wherein the pH of the feed solution has a pH in the range of about 5 to about 10; and b) compressing the obtained powder of GLP-1 peptide and optionally at least one pharmaceutically acceptable excipient into a tablet.

In some embodiments, the process for producing a tablet comprises the steps of a) providing the GLP-1 peptide in a feed solution; b) adjusting the pH of the feed solution to a pH above the pl of the GLP-1 peptide or to a pH in the range of about 5 to about 10 by addition of a non-volatile base to the feed solution; c) introducing the feed solution into a spray-dryer and drying said GLP-1 peptide; d) compressing the obtained GLP-1 powder and optionally at least one pharmaceutically acceptable excipient into a tablet.

A 'non-volatile' base as mentioned herein is a base, which does not evaporate during a spray-drying process. Exemplary non-volatile bases are sodium hydroxide, potassium hydroxide, buffers selected from e.g. a phosphate buffer, a TRIS buffer, an acetate buffer. The 'pl' as used herein refers to the isoelectric point of the GLP-1 peptide, i.e. the pH value where the peptide carried no net charge.

In some embodiments, the feed solution comprising the GLP-1 peptide is obtained by solubilising a powder of the GLP-1 peptide in the feed solution solvent. Also or alternatively, In some embodiments, the feed solution is directly obtained from a previous manufacturing step, e.g. the eluent of a final chromatographic purification, from ultrafiltration, or from diafiltration. In some embodiments, the feed solution from a previous manufacturing step comprises the GLP-1 peptide as main solid component (80-100 percent (w/w) of solid components), but may also comprise smaller amounts of salts and impurities carried over from manufacturing.

In some embodiments, the pH of the feed solution comprising the GLP-1 peptide is in the range of pH of about 4 to about 12, such as about 5 to about 10, about 6 to about 9 or about 6 to about 8. In some embodiments, the pH of the feed solution comprising the GLP-1 peptide is in the range of pH of 4-12, such as 5-10, 6-9 or 6-8. Also or alternatively, In some embodiments, the pH pf the feed solution comprising the GLP-1 peptide has a pH higher than pl of the GLP-1 peptide. In some embodiments, the pH of the feed solution comprising the GLP-1 peptide is in the range of pH of about 5 to about 10. In some embodiments the pH of the feed solution comprising the GLP-1 peptide is about 5 to about 9, such as about 6 to about 8, about 7 to about 9 or about 8 to about 10.

In some embodiments, the term "about" as used herein means ±10% of the value referred to, and includes the value.

In some embodiments, the pH of the feed solution is adjusted by addition of a non-volatile base such as an aqueous 1M sodium hydroxide solution. pH is measured at room temperature using either a probe or strips in the feed solution solvent, e.g. an aqueous alcoholic solvent, such as aqueous ethanol.

In some embodiments, the feed solution comprises a GLP-1 peptide in a feed solution solvent. In some embodiments, the GLP-1 peptide is selected from semaglutide or Nε27-[2-[2-[2-[[2-[2-[2-[[(4S)-4-carboxy-4-[10-(4-carboxyphenoxy)decanoylamino]butanoyl]amino]ethoxy] ethoxy]acetyl]amino]ethoxy]ethoxy]acetyl], Nε36-[2-[2-[2-[[2-[2-[2-[[(4S)-4-carboxy-4-[10-(4-carboxyphenoxy)decanoylamino]butanoyl]amino]ethoxy]ethoxy]acetyl]amino]ethoxy]ethoxy]a cetyl]-[Aib8,Glu22,Arg26,Lys27,Glu30,Arg34,Lys36]-GLP-1-(7-37)-peptidyl-Glu-Gly (Compound A).

In some embodiments, the pH of the feed solution comprising semaglutide is above about 5.4. In some embodiments, the pH of the feed solution comprising semaglutide is above 5.4. In some embodiments, the pH of the feed solution comprising semaglutide is in the range of about 5 to about 12, such as about 5.4 to about 12, about 6 to about 10 or about 6 to about 8. In some embodiments, the pH of the feed solution comprising semaglutide is in the range of 5-12, such as 5.4-12, 6-10 or 6-8. In some embodiments,
In some embodiments, the pH of the feed solution comprising Compound A is above about 4.6. In some embodiments, the pH of the feed solution comprising Compound A is above 4.6. In some embodiments, the pH of the feed solution comprising Compound A is in the range of about 4.6 to about 12, such as about 6 to about 10, about 7 to about 10 or about 7 to about 9. In some embodiments, the pH of the feed solution comprising Compound A is in the range of 4.6-12, such as 6-10 or 7-9. In some embodiments, the pH of the feed solution comprising Compound A is in the range of about 8 to about 10, such as about 8.5 to about 9.5.

In some embodiments, the feed solution solvent used for spray-drying is a mixture of a water miscible organic solvent and water. A 'water miscible organic solvent' as used herein refers to an organic solvent that upon mixture with water will result in a homogeneous solution, e.g. alcoholic solvents such as methanol, ethanol, isopropanol, acetone, or acetonitrile. In some embodiments, the feed solution solvent is an aqueous alcoholic solvent, such as aqueous ethanol, i.e. comprising water and ethanol. In some embodiments, the feed solution consists essentially of the GLP-1 peptide in aqueous ethanol. In some embodiments, the aqueous ethanol is in a concentration of about 40 to about 70 percent (w/w), such as about 45 to about 65 percent (w/w) or about 50 to about 60 percent (w/w). In some embodiments, the aqueous ethanol is in a concentration of 40-70 percent (w/w), such as 45-65 percent (w/w) or 50-60 percent (w/w). The concentration of aqueous ethanol is defined from the content of ethanol, i.e. about 70 percent (w/w) aqueous ethanol consists substantially of about 70 percent ethanol and about 30 percent water by weight. The concentration of aqueous ethanol is defined from the content of ethanol, i.e. 70 percent (w/w) aqueous ethanol consist substantially of 70 percent ethanol and 30 percent water by weight.

In some embodiments, the concentration of the GLP-1 peptide in the feed solution for spray-drying is about 0.5 to about 10 percent (w/w), such as about 1 to about 7 percent (w/w), about 1 to about 5 percent (w/w) or about 1 to about 3 percent (w/w). In some embodiments, the concentration of the GLP-1 peptide in the feed solution for spray-drying is 0.5-10 percent (w/w), such as 1-7 percent (w/w), 1-5 percent (w/w) or 1-3 percent (w/w). In a particular embodiment, the concentration of the GLP-1 peptide in the feed solution is about 1.8 to about 2.1 percent (w/w) e.g. in about 50 to about 55 percent (w/w) aqueous ethanol, such as about 1.8 to about 2.1 percent (w/w) semaglutide in about 50 to about 55 percent (w/w) aqueous ethanol. In a particular embodiment, the concentration of the GLP-1 peptide in the feed solution is 1.8-2.1 percent (w/w) e.g. in 50-55 percent (w/w) aqueous ethanol, such as 1.8-2.1 percent (w/w) semaglutide in 50-55 percent (w/w) aqueous ethanol. Also or alternatively, in one embodiment, the concentration of the GLP-1 peptide in the feed solution is about 3 to about 5 percent (w/w) e.g. in about 50 to about 65 percent (w/w) aqueous ethanol, such as about 3 to about 5 percent (w/w) of Compound A in about 50 to about 65 percent (w/w) aqueous ethanol. Also or alternatively, in one embodiment, the concentration of the GLP-1 peptide in the feed solution is 3-5 percent (w/w) e.g. in 50-65 percent (w/w) aqueous ethanol, such as 3-5 percent (w/w) of Compound A in 50-65 percent (w/w) aqueous ethanol.

In some embodiments, the process comprises a compression step, wherein the powder of GLP-1 peptide obtained from spray-drying step a) is compressed into a tablet, optionally further comprising at least one pharmaceutically acceptable excipient. In some embodiments, the tablet produced comprises a therapeutically effective amount of the GLP-1 peptide. In some embodiments, the tablet produced comprises the GLP-1 peptide in an amount of about 0.01 to about 100 mg. In some embodiments, the tablet produced comprises the GLP-1 peptide in an amount of 0.01-100 mg. In some embodiments, the tablet produced comprises about 0.1 to about 80 mg or about 1 to about 30 mg of the GLP-1 peptide. In some embodiments, the tablet produced comprises 0.1-80 mg or 1-30 mg of the GLP-1 peptide.

### Process for producing a GLP-1 peptide powder

In some embodiments, the invention relates to a process for producing a spray-dried powder of GLP-1 peptide, wherein the GLP-1 peptide is semaglutide or Compound A, comprising the step of spray-drying a feed solution comprising the GLP-1 peptide and a feed solution solvent, wherein the pH of the feed solution is higher than the pl of the GLP-1 peptide or wherein the pH of the feed solution is in the range of about 5 to about 10. In some embodiments, the process for producing a spray-dried powder of GLP-1 peptide, wherein the GLP-1 peptide is semaglutide or Compound A, comprises the steps of a) providing the GLP-1 peptide in a feed solution; b) adjusting the pH of the feed solution to a pH above the pl of the GLP-1 peptide or to a pH in the range of about 5 to about 10 by addition of a non-volatile base to the feed solution; c) introducing the feed solution into a spray-dryer and drying said GLP-1 peptide. In some embodiments, the feed solution comprising the GLP-1 peptide is obtained by solubilising a powder of the GLP-1 peptide in the feed solution solvent. Also or alternatively, In some embodiments, the feed solution is directly obtained from a previous manufacturing step, e.g. the eluent of a final chromatographic purification, from ultrafiltration, or from diafiltration. In some embodiments, the feed solution from a previous manufacturing step comprises the GLP-1 peptide as main solid component (80-100 percent (w/w) of solid components), but may also comprise smaller amounts of salts and impurities carried over from manufacturing.

In some embodiments, the pH of the feed solution comprising the GLP-1 peptide is in the range of pH of about 4 to about 12, such as about 5 to about 10, about 6 to about 9 or about 6 to about 8. Also or alternatively, In some embodiments, the pH pf the feed solution comprising the GLP-1 peptide has a pH higher than pl of the GLP-1 peptide. In some embodiments, the pH of the feed solution comprising the GLP-1 peptide is in the range of pH of about 5 to about 10. In some embodiments the pH of the feed solution comprising the GLP-1 peptide is about 5 to about 9, such as about 6 to about 8, about 7 to about 9 or about 8 to about 10.In some embodiments, the pH of the feed solution comprising the GLP-1 peptide is in the range of about 8 to about 10.

In some embodiments, the pH of the feed solution is adjusted by addition of a non-volatile base such as an aqueous 1M sodium hydroxide solution. pH is measured at room temperature using either a probe or strips in the feed solution solvent, e.g. an aqueous alcoholic solvent, such as aqueous ethanol.

In some embodiments, the pH of the feed solution comprising semaglutide is above about 5.4. In some embodiments, the pH of the feed solution comprising semaglutide is in the range of about 5 to about 12, such as about 5.4 to about 12, about 6 to about 10 or about 6 to about 8.

In some embodiments, the pH of the feed solution comprising Compound A is above about 4.6. In some embodiments, the pH of the feed solution comprising Compound A is in the range of about 4.6 to about 12, such as about 6 to about 10 or about 7 to about 9. In some embodiments, the pH of the feed solution comprising semaglutide is in the range of about 8 to about 10, such as about 8.5 to about 9.5.

In some embodiments, the feed solution solvent used for spray-drying is a mixture of a water miscible organic solvent and water. In some embodiments, the feed solution solvent is an aqueous alcoholic solvent, such as aqueous ethanol, i.e. comprising water and ethanol. In some embodiments, the feed solution consists essentially of the GLP-1 peptide in aqueous ethanol. In some embodiments, the aqueous ethanol is in a concentration of about 40-70 percent (w/w), such as about 45 to about 65 percent (w/w) or about 50 to about 60 percent (w/w). The concentration of aqueous ethanol is defined from the content of ethanol, i.e. about 70 percent (w/w) aqueous ethanol consist substantially of about 70 percent ethanol and about 30 percent water by weight.

In some embodiments, the concentration of the GLP-1 peptide in the feed solution for spray-drying is about 0.5 to about 10 percent (w/w), such as about 1 to about 7 percent (w/w), about 1 to about 5 percent (w/w) or about 1 to about 3 percent (w/w). In a particular embodiment, the concentration of the GLP-1 peptide in the feed solution is about 1.8 to about 2.1 percent (w/w) e.g. in about 50 to about 55 percent (w/w) aqueous ethanol, such as about 1.8 to about 2.1 percent (w/w) semaglutide in about 50 to about 55 percent (w/w) aqueous ethanol. Also or alternatively, in one embodiment, the concentration of the GLP-1 peptide in the feed solution is 3-5 percent (w/w) e.g. in about 50 to about 65 percent (w/w) aqueous ethanol, such as about 3 to about 5 percent (w/w) of Compound A in about 50 to about 65 percent (w/w) aqueous ethanol.

In some embodiments, the GLP-1 peptide powder as described herein may have an intrinsic dissolution rate at 25°C of about 1 mg/(min*cm²), such as 1.0 mg/(min*cm²), or 1.1 mg/(min*cm²), or 1.4 mg/(min*cm²), or 1.5 mg/(min*cm²). In some embodiments, the GLP-1 peptide powder as described herein may have an intrinsic dissolution rate at 25°C of about 2 mg/(min*cm²), such as 2.6 mg/(min*cm²) or 2.1 mg/(min*cm²). In some embodiments, the GLP-1 peptide powder as described herein may have an intrinsic dissolution rate at 25°C of about 4 mg/(min*cm²), such 4.2 mg/(min*cm²). In some embodiments, the GLP-1 peptide powder as described herein may have an intrinsic dissolution rate at 25°C of about 6 mg/(min*cm²), such 6.4 mg/(min*cm²). In some embodiments, the GLP-1 peptide powder as described herein may have an intrinsic dissolution rate at 30 °C of about 1 mg/(min*cm²), such as 1.0 mg/(min*cm²), or 1.1 mg/(min*cm²), or 1.4 mg/(min*cm²), or 1.5 mg/(min*cm²). In some embodiments, the GLP-1 peptide powder as described herein may have an intrinsic dissolution rate at 30 °C of about 2 mg/(min*cm²), such as 2.6 mg/(min*cm²) or 2.1 mg/(min*cm²). In some embodiments, the GLP-1 peptide powder as described herein may have an intrinsic dissolution rate at 30 °C of about 4 mg/(min*cm²), such 4.2 mg/(min*cm²). In some embodiments, the GLP-1 peptide powder as described herein may have an intrinsic dissolution rate at 30 °C of about 6 mg/(min*cm²), such 6.4 mg/(min*cm²). In some embodiments, the GLP-1 peptide powder is semaglutide powder or compound A powder. In some embodiments, the semaglutide powder has an intrinsic dissolution rate at 25°C of about 1 mg/(min*cm²), such as 1.0 mg/(min*cm²), or 1.1 mg/(min*cm²), or 1.4 mg/(min*cm²), or 1.5 mg/(min*cm²). In some embodiments, the compound A powder has an intrinsic dissolution rate 25 °C of above 2 mg/(min*cm²). In some embodiments, the compound A powder has an intrinsic dissolution rate 30 °C in the range of 2-7 mg/(min*cm²), such as 2.1-7, 2.6-7, 4.2 or 6.4 mg/(min*cm²). The intrinsic dissolution rate [mg/(min*cm²)] is defined as the rate of dissolution of a pharmaceutical active substance formulated into a compact when conditions such as the total exposed surface area of the compact as well as the temperature, agitationstirring speed, pH, and ionic strength of the dissolution medium are kept constant.

### Pharmaceutical compositions

In some embodiments, the invention relates to a pharmaceutical composition comprising a GLP-1 peptide powder according to the invention, and optionally one or more pharmaceutically-acceptable excipients. The term 'excipient' as used herein broadly refers to any component other than the active therapeutic ingredient(s). The excipient may be an inert substance, an inactive substance, and/or a not medicinally active substance. The excipient may serve various purposes, e.g. as a carrier, vehicle, filler, binder, lubricant, glidant, disintegrant, flow control agents, crystallization retarders, solubilizers, stabilizer, colouring agent, flavouring agent, surfactant, emulsifier and/or to improve administration, and/or absorption of the active substance, e.g. a delivery agent/absorption enhancer. A person skilled in the art may select one or more of the aforementioned excipients with respect to the particular desired properties of the solid oral dosage form by routine experimentation and without any undue burden. The amount of each excipient used may vary within ranges conventional in the art. Techniques and excipients which may be used to formulate oral dosage forms are described in Handbook of Pharmaceutical Excipients, 6th edition, Rowe et al., Eds., American Pharmaceuticals Association and the Pharmaceutical Press, publications department of the Royal Pharmaceutical Society of Great Britain (2009); and Remington: the Science and Practice of Pharmacy, 21th edition, Gennaro, Ed., Lippincott Williams and Wilkins (2005). In some embodiments, the excipients may be selected from binders, such as polyvinyl pyrrolidone (povidone), etc.; fillers such as cellulose powder, microcrystalline cellulose, cellulose derivatives like hydroxymethylcellulose, hydroxyethylcellulose, hydroxypropylcellulose and hydroxy-propylmethylcellulose, dibasic calcium phosphate, corn starch, pregelatinized starch, etc.; lubricants and/or glidants such as stearic acid, magnesium stearate, sodium stearylfumarate, glycerol tribehenate, etc.; flow control agents such as colloidal silica, talc, etc.; crystallization retarders such as povidone, etc.; solubilizers such as Pluronic, povidone, etc.; colouring agents, including dyes and pigments such as Iron Oxide Red or Yellow, titanium dioxide, talc, etc.; pH control agents such as citric acid, tartaric acid, fumaric acid, sodium citrate, dibasic calcium phosphate, dibasic sodium phosphate, etc.; surfactants and emulsifiers such as Pluronic, polyethylene glycols, sodium carboxymethyl cellulose, polyethoxylated and hydrogenated castor oil, etc.; and mixtures of two or more of these excipients and/or adjuvants.

In some embodiments, a pharmaceutical composition as described herein may comprise a salt of N-(8-(2-hydroxybenzoyl)amino)caprylic acid and/or a lubricant, such as magnesium stearate, and/or a filler, such as microcrystalline cellulose, and/or a binder, such a povidone.

In some embodiments, the pharmaceutical composition may comprise a first type of granules and a second type of granules, wherein the first type of granules comprises a salt of N-(8-(2-hydroxybenzoyl)amino)caprylic acid and the second type of granule comprises a GLP-1 peptide powder according to the invention. In some embodiments the first type of granules further comprises a lubricant, such as magnesium stearate. In some embodiments the first type of granules further comprises a filler, such as microcrystalline cellulose. Accordingly, the first type of granules may further comprise a lubricant and optionally a filler. In some embodiments the second type of granules further comprises a filler, such as microcrystalline cellulose. In some embodiments the second type of granules further comprises a binder, such as povidone. Accordingly, the second type of granules may further comprise a filler and optionally a binder. In some embodiments the composition further comprises an extragranular lubricant, such as magnesium stearate.

In some embodiments, the composition is in the form of a solid dosage form. In some embodiments the composition is in the form of a tablet. In some embodiments the composition is in form of a capsule. In some embodiments the composition is in form of a sachet.

For the avoidance of doubt, references herein to GLP-1 peptide powder of the invention being for particular uses (and, similarly, to uses and methods of use relating to compounds of the invention) may also apply to pharmaceutical compositions comprising compounds of the invention, as described herein.

### Oral dosage forms

In some embodiments, the invention relates to an oral dosage form comprising a GLP-1 peptide powder obtainable by the process of the invention. In some embodiments, the oral dosage form is a tablet, capsule or sachet. In some embodiments, the oral dosage form is a sachet. In some embodiments, the oral dosage form is a capsule. In some embodiments the oral dosage form is a tablet.

### Tablet

In some embodiments, the invention relates to a tablet obtained by the process as described in paragraph 'Process for producing a tablet'. In some embodiments, the invention relates to a tablet comprising a GLP-1 peptide, wherein the GLP-1 peptide is obtained by a spray-drying process wherein a feed solution comprising the GLP-1 peptide and a feed solution solvent, wherein the pH of the feed solution is higher than the pl of the GLP-1 peptide or wherein the pH of the feed solution is in the range of about 5 to about 10. In some embodiments, the tablet comprising a GLP-1 peptide is obtained by the spray-drying process comprising the steps of a) providing the GLP-1 peptide in a feed solution; b) adjusting the pH of the feed solution to a pH above the pl of the GLP-1 peptide or to a pH in the range of about 5 to about 10 by addition of a non-volatile base to the feed solution; c) introducing the feed solution into a spray-dryer and drying said GLP-1 peptide. The GLP-1 peptide obtained by the spray-drying process as described is subsequently compressed into a tablet optionally with at least one pharmaceutically acceptable excipient.

In some embodiments, the tablet of the invention comprising a GLP-1 peptide is obtained by spray-drying using a feed solution wherein the pH of the feed solution comprising the GLP-1 peptide is in the range of pH of about 5 to about 12, such as about 5 to about 10 or about 6 to about 8. In some embodiments, the tablet of the invention comprising a GLP-1 peptide is obtained by spray-drying using a feed solution wherein the pH of the feed solution comprising the GLP-1 peptide is in the range of pH of 5-12, such as 5-10 or 6-8. Also or alternatively, In some embodiments, the GLP-1 peptide comprised in the tablet, is obtained by spray-drying a feed solution wherein the pH of the feed solution comprising the GLP-1 peptide has a pH higher than pl of the GLP-1 peptide or a pH in the range of about 5 to about 10. In some embodiments, the pH of the feed solution is adjusted by addition of a non-volatile base e.g. aqueous sodium hydroxide in a concentration of e.g. 1M.

In some embodiments, the tablet of the invention comprises a GLP-1 peptide powder obtained by the process of the invention wherein the GLP-1 peptide powder has an intrinsic dissolution rate at 25 °C of about 1 mg/(min*cm²), such as 1.0 mg/(min*cm²), or 1.1 mg/(min*cm²), or 1.4 mg/(min*cm²), or 1.5 mg/(min*cm²). In some embodiments, the tablet of the invention comprises a GLP-1 peptide powder obtained by the process of the invention wherein the GLP-1 peptide powder has an intrinsic dissolution rate at 25 °C of about 2 mg/(min*cm²), such as 2.6 mg/(min*cm²) or 2.1 mg/(min*cm²). In some embodiments, the tablet of the invention comprises a GLP-1 peptide powder obtained by the process of the invention wherein the GLP-1 peptide powder has an intrinsic dissolution rate at 25°C of about 4 mg/(min*cm²), such 4.2 mg/(min*cm²). In some embodiments, the tablet of the invention comprises a GLP-1 peptide powder obtained by the process of the invention wherein the GLP-1 peptide powder has an intrinsic dissolution rate at 25°C of about 6 mg/(min*cm²), such 6.4 mg/(min*cm²). In some embodiments, the tablet of the invention comprises a GLP-1 peptide powder obtained by the process of the invention wherein the GLP-1 peptide powder has an intrinsic dissolution rate at 30 °C of about 1 mg/(min*cm²), such as 1.0 mg/(min*cm²), or 1.1 mg/(min*cm²), or 1.4 mg/(min*cm²), or 1.5 mg/(min*cm²). In some embodiments, the tablet of the invention comprises a GLP-1 peptide powder obtained by the process of the invention wherein the GLP-1 peptide powder has an intrinsic dissolution rate at 30 °C of about 2 mg/(min*cm²), such as 2.6 mg/(min*cm²) or 2.1 mg/(min*cm²). In some embodiments, the tablet of the invention comprises a GLP-1 peptide powder obtained by the process of the invention wherein the GLP-1 peptide powder has an intrinsic dissolution rate at 30 °C of about 4 mg/(min*cm²), such 4.2 mg/(min*cm²). In some embodiments, the tablet of the invention comprises a GLP-1 peptide powder obtained by the process of the invention wherein the GLP-1 peptide powder has an intrinsic dissolution rate at 30 °C of about 6 mg/(min*cm²), such 6.4 mg/(min*cm²).

In some embodiments, the tablet comprises a GLP-1 peptide selected from semaglutide or Nε27-[2-[2-[2-[[2-[2-[2-[[(4S)-4-carboxy-4-[10-(4-carboxyphenoxy)decanoylamino]butanoyl]amino]ethoxy]ethoxy] acetyl]amino]ethoxy]ethoxy]acetyl], Nε36-[2-[2-[2-[[2-[2-[2-[[(4S)-4-carboxy-4-[10-(4-carboxyphenoxy)decanoylamino]butanoyl]amino]ethoxy]ethoxy]acetyl]amino]ethoxy]ethoxy]a cetyl]-[Aib8,Glu22,Arg26,Lys27,Glu30,Arg34,Lys36]-GLP-1-(7-37)-peptidyl-Glu-Gly (Compound A).

In some embodiments, the tablet comprises a therapeutically effective amount of the GLP-1 peptide. In some embodiments, the tablet comprises the GLP-1 peptide in an amount of about 0.01 to about 100 mg. In some embodiments, the tablet comprises the GLP-1 peptide in an amount of 0.01-100 mg. In some embodiments, the tablet comprises about 0.2 to about 80 mg, about 0.5 to about 60 mg or about 1 to about 30 mg of the GLP-1 peptide. In some embodiments, the tablet comprises 0.2-80 mg, 0.5-60 mg or 1-30 mg of the GLP-1 peptide.

In some embodiments, the tablet is a solid composition compressed into a tablet and is intended for oral administration.

The tablet may comprise at least one pharmaceutically acceptable excipient.

In some embodiments, the tablet comprises at least about 60 percent (w/w) of a delivery agent, such as at least about 70 percent (w/w) or at least about 75 percent (w/w). In some embodiments, the tablet comprises at least 60 percent (w/w) of a delivery agent, such as at least 70 percent (w/w) or at least 75 percent (w/w). In some embodiments, the delivery agent is a salt of N-(8-(2-hydroxybenzoyl)amino)caprylic acid, such as sodium N-(8-(2-hydroxybenzoyl)amino caprylate. In some embodiments, the delivery agent is a salt of N-(8-(2-hydroxybenzoyl)amino)caprylic acid, e.g. sodium N-(8-(2-hydroxybenzoyl)amino caprylate, and constitutes at least about 90 percent (w/w) of the total amount of excipients in the tablet. In some embodiments, the delivery agent is a salt of N-(8-(2-hydroxybenzoyl)amino)caprylic acid, e.g. sodium N-(8-(2-hydroxybenzoyl)amino caprylate, and constitutes at least 90 percent (w/w) of the total amount of excipients in the tablet.

In some embodiments, the tablet comprises about 0.1 to about 10 percent (w/w) of lubricant and/or glidant or about 0.5 to about 5 percent (w/w), such as about 1 to about 3.5 percent (w/w) or about 1 percent (w/w). In some embodiments, the tablet comprises 0.1-10 percent (w/w) of lubricant and/or glidant or 0.5-5 percent (w/w), such as 1-3.5 percent (w/w) or 1 percent (w/w). In some embodiments, the tablet comprises about 2 to about 2.5 percent (w/w). In some embodiments, the tablet comprises a lubricant and/or a glidant, such as a salt of stearic acid. In some embodiments, the tablet comprises magnesium stearate as lubricant. In some embodiments, the lubricant is magnesium stearate and constitutes no more than about 10 percent (w/w) of the total amount of excipients of the tablet. In some embodiments, the tablet comprises magnesium stearate as lubricant. In some embodiments, the lubricant is magnesium stearate and constitutes no more than 10 percent (w/w) of the total amount of excipients of the tablet.

Still further, the tablet may be formulated as is known in the art of oral formulations of insulinotropic compounds, e.g. using any one or more of the formulations described in WO 2008/145728 and WO/2019/149880.

A tablet may also be used in the formulation of site specific, controlled, sustained, protracted, prolonged, delayed, pulsatile, retarded, and/or slow release drug delivery systems.

The tablet obtained by the process of the invention may be prepared as is known in the art. A tablet press may be used to compress the "compacting material" or the "tabletting material". By "compacting material" we mean the GLP-1 peptide obtained from spray-drying as described herein. By "tabletting material" we mean the GLP-1 peptide obtained from spray-drying as described herein and the at least one pharmaceutically acceptable excipient. In some embodiments, the ingredients are granulated prior to being compressed or compacted into a tablet. In some embodiments, the tablet is compacted using a tablet press. In a tabletting press, the tabletting material is filled (e.g. force fed or gravity fed) into a die cavity. The tabletting material is then compacted by a punch with pressure. Subsequently, the resulting compact, or tablet is ejected from the tabletting press. The above-mentioned compaction process is subsequently referred to herein as the "compaction process". Suitable tablet presses include, but are not limited to, rotary tablet presses and eccentric tablet presses. Examples of tablet presses include, but are not limited to, the Fette 102i (Fette GmbH), the Korsch XL100, the Korsch PH 106 rotary tablet press (Korsch AG, Germany), the Korsch EK-0 eccentric tabletting press (Korsch AG, Germany) and the Manesty F-Press (Manesty Machines Ltd., United Kingdom).

In some embodiments, the GLP-1 peptide powder as described herein may be further granulated. The term "granule" may refer to particles gathered into larger particles. A "granule" may be formed by gathering small particles into a large mass. The term "granulate" refers to several granules, such as two or more granules. If the GLP-1 peptide powder as described herein is to be manufactured into granules and such granules are to be used in the tabletting material, said granules may be produced in a manner known to a person skilled in the art, for example by dry granulation techniques in which the pharmaceutically active agent and/or delivery agents are compacted with the excipients to form relatively large moldings, for example slugs or ribbons, which are comminuted by grinding, and the ground material serves as the tabletting material to be later compressed into tablets. Suitable equipment for dry granulation includes but is not limited to roller compaction equipment from Gerteis, such as Gerteis MINI-PACTOR.

The tablet may be administered in several dosage forms, for example as an uncoated or coated tablet.

The tablet produced by the process of the invention may comprise at least one pharmaceutically acceptable excipient. A person skilled in the art may select one or more of the aforementioned excipients with respect to the particular desired properties of the solid oral dosage form by routine experimentation and without any undue burden. The amount of each excipient used may vary within ranges conventional in the art.

The tablet may be of a suitable weight. In some embodiments, the weight of the tablet is in the range of about 100 mg to about 1000 mg, such as in the range of about 175 mg to about 1000 mg or such as about 100 mg. In some embodiments, the weight of the tablet is in the range of about 175 to about 250 mg, about 300 to about 500 mg or about 500 to about 900 mg. In some embodiments, the weight of the tablet is in the range of 175 mg to 1000 mg, such as in the range of 175- 250 mg, 300-500 mg or 500-900 mg, or such as about 200 mg, about 400 mg or about 700 mg. In some embodiments, the weight of the tablet is in the range of about 200 mg to about 1000 mg, such as in the range of about 500 to about 700 mg or about 600 to about 1000 mg. In some embodiments, the weight of the tablet is in the range of 200 mg to 1000 mg, such as in the range of 500-700 mg or 600-1000 mg, or such as about 200 mg, about 400 mg, about 600 mg or about 800 mg.

### GLP-1 peptides

The tablet obtained by the process of the invention comprises a GLP-1 peptide. The term 'GLP-1 peptide' as used herein refers to a peptide that fully or partially activates the human GLP-1 receptor.

In some embodiments, the GLP-1 peptide may be semaglutide. Semaglutide may be prepared as described in WO2006/097537, Example 4. Semaglutide is also known as N^{6,26}-{18-[N-(17-carboxyheptadecanoyl)-L-γ-glutamyl]-10-oxo-3,6,12,15-tetraoxa-9,18-diazaoctadecanoyl}-[8-(2-amino-2-propanoic acid), 34-L-arginine]human glucagon-like peptide 1(7-37), see WHO Drug Information Vol. 24, No. 1, 2010. pl of semaglutide is about 5.4. Semaglutide has the following structure:

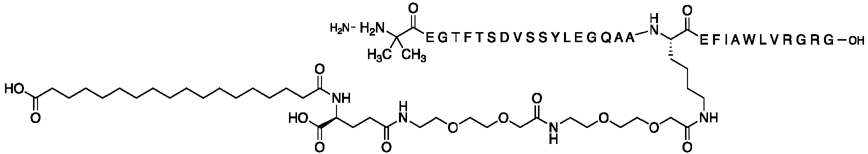

In some embodiments, the GLP-1 peptides may be found in WO 2012/140117, e.g. Nε27-[2-[2-[2-[[2-[2-[2-[[(4S)-4-carboxy-4-[10-(4-carboxyphenoxy)decanoylamino]butanoyl]amino]ethoxy]ethoxy] acetyl]amino]ethoxy]ethoxy]acetyl], Nε36-[2-[2-[2-[[2-[2-[2-[[(4S)-4-carboxy-4-[10-(4-carboxyphenoxy)decanoylamino]butanoyl]amino]ethoxy]ethoxy]acetyl]amino]ethoxy]ethoxy]a cetyl]-[Aib8,Glu22,Arg26,Lys27,Glu30,Arg34,Lys36]-GLP-1-(7-37)-peptidyl-Glu-Gly (Compound A) and may be prepared as described in WO2012/140117, example 31. pl of Compound A is about 4.6. Compound A has the following structure

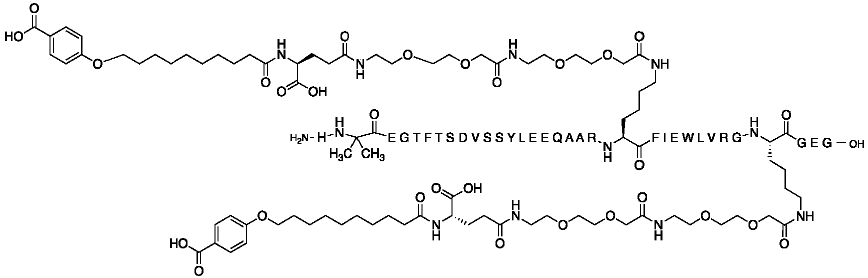

In some embodiments, the GLP-1 peptide may be present in its fully or partly ionised form; for example one or more carboxylic acid groups (-COOH) may be deprotonated into the
carboxylate group (-COO⁻) and/or one or more amino groups (-NH₂) may be protonated into the -NH₃⁺ groups. In some embodiments, the GLP-1 peptide is in the form of a salt.

### Pharmaceutical Indications

The present invention relates to an oral dosage form such as tablet of the invention or pharmaceutical composition of the invention for use as a medicament. In particular embodiments, the tablet may be used in the following medical treatments, all preferably relating to diabetes:
(i) prevention and/or treatment of all forms of diabetes, such as hyperglycemia, type 2 diabetes, impaired glucose tolerance, type 1 diabetes, non-insulin dependent diabetes, MODY (maturity onset diabetes of the young), gestational diabetes, and/or for reduction of HbA1C;
(ii) delaying or preventing diabetic disease progression, such as progression in type 2 diabetes, delaying the progression of impaired glucose tolerance (IGT) to insulin requiring type 2 diabetes, and/or delaying the progression of non-insulin requiring type 2 diabetes to insulin requiring type 2 diabetes;
(iii) improving beta -cell function, such as decreasing beta -cell apoptosis, increasing beta -cell function and/or beta -cell mass, and/or for restoring glucose sensitivity to beta -cells;
(iv) prevention and/or treatment of cognitive disorders;
(v) prevention and/or treatment of eating disorders, such as obesity, e.g. by decreasing food intake, reducing body weight, suppressing appetite, inducing satiety; treating or preventing binge eating disorder, bulimia nervosa, and/or obesity induced by administration of an antipsychotic or a steroid; reduction of gastric motility; and/or delaying gastric emptying;
(vi) prevention and/or treatment of diabetic complications, such as neuropathy, including peripheral neuropathy; nephropathy; or retinopathy;
(vii) improving lipid parameters, such as prevention and/or treatment of dyslipidemia, lowering total serum lipids; lowering HDL; lowering small, dense LDL; lowering VLDL: lowering triglycerides; lowering cholesterol; increasing HDL; lowering plasma levels of lipoprotein a (Lp(a)) in a human; inhibiting generation of apolipoprotein a (apo(a)) in vitro and/or in vivo;
(viii) prevention and/or treatment of cardiovascular diseases, such as syndrome X; atherosclerosis; myocardial infarction; coronary heart disease; stroke, cerebral ischemia; an early cardiac or early cardiovascular disease, such as left ventricular hypertrophy; coronary artery disease; essential hypertension; acute hypertensive emergency; cardiomyopathy; heart insufficiency; exercise tolerance; chronic heart failure; arrhythmia; cardiac dysrhythmia; syncopy; atheroschlerosis; mild chronic heart failure; angina pectoris; cardiac bypass reocclusion; intermittent claudication (atheroschlerosis oblitterens); diastolic dysfunction; and/or systolic dysfunction;
(ix) prevention and/or treatment of gastrointestinal diseases, such as inflammatory bowel syndrome; small bowel syndrome, or Crohn's disease; dyspepsia; and/or gastric ulcers;
(x) prevention and/or treatment of critical illness, such as treatment of a critically ill patient, a critical illness poly-nephropathy (CIPNP) patient, and/or a potential CIPNP patient; prevention of critical illness or development of CIPNP; prevention, treatment and/or cure of systemic inflammatory response syndrome (SIRS) in a patient; and/or for the prevention or reduction of the likelihood of a patient suffering from bacteraemia, septicaemia, and/or septic shock during hospitalisation; and/or
(xi) prevention and/or treatment of polycystic ovary syndrome (PCOS).

In a particular embodiment, the indication is selected from the group consisting of (i)-(iii) and (v)-(viii), such as indications (i), (ii), and/or (iii); or indication (v), indication (vi), indication (vii), and/or indication (viii). In another particular embodiment, the indication is (i). In a further particular embodiment, the indication is (v). In a still further particular embodiment the indication is (viii). In some embodiments, the indications are type 2 diabetes and/or obesity.

Unless otherwise indicated in the specification, terms presented in singular form also include the plural form.

In some embodiments, as used herein, specific values given in relation to numbers or intervals may be understood as the specific value or as about the specific value (e.g. plus or minus 10 percent of the specific value).

### Embodiments

The following are non-limiting embodiments of the invention:
1. A process of producing a tablet comprising a GLP-1 peptide, said method comprising the steps of:
   a. Spray-drying a feed solution comprising the GLP-1 peptide and a feed solution solvent, wherein the pH of the feed solution is higher than the pl of the GLP-1 peptide;
   b. Compressing the obtained powder of GLP-1 peptide and optionally at least one pharmaceutically acceptable excipient into a tablet.
2. The process according to embodiment 1 comprising the steps of:
   a. Dissolving the GLP-1 peptide in the feed solution solvent or obtaining the GLP-1 peptide in solution directly from a purification step;
   b. Adjusting pH of the feed solution to a pH above pl of the GLP-1 peptide by addition of a non-volatile base to the feed solution;
   c. Introducing the feed solution into a spray-dryer and drying said GLP-1 peptide;
   d. Compressing the obtained powder of GLP-1 peptide and optionally at least one pharmaceutically acceptable excipient into a tablet.
3. A process of producing a tablet comprising a GLP-1 peptide, said method comprising the steps of:
   a. Spray-drying a feed solution comprising the GLP-1 peptide and a feed solution solvent, wherein the pH of the feed solution is in the range 5-12;
   b. Compressing the obtained powder of GLP-1 peptide and optionally at least one pharmaceutically acceptable excipient into a tablet.
4. The process according to embodiment 3 comprising the steps of:
   a. Dissolving the GLP-1 peptide in the feed solution solvent or obtaining the GLP-1 peptide in solution directly from a purification step;
   b. Adjusting pH of the feed solution to pH 5-12 by addition of a non-volatile base to the feed solution;
   c. Introducing the feed solution into a spray-dryer and drying said GLP-1 peptide;
   d. Compressing the obtained powder of GLP-1 peptide and optionally at least one pharmaceutically acceptable excipient into a tablet.
5. A process of producing a spray-dried powder of a GLP-1 peptide powder, wherein the GLP-1 peptide is semaglutide or Compound A, the process comprising the step or spray-drying a feed solution comprising the GLP-1 peptide and a feed solution solvent, wherein the pH of the feed solution is in the range of about 5 to about 10.
6. The process according to embodiment 5, comprising the steps of:
   a. Dissolving the GLP-1 peptide in the feed solution solvent or obtaining the GLP-1 peptide in solution directly from a purification step;
   b. Adjusting pH of the feed solution to a pH in the range of about 5 to about 10 by addition of a non-volatile base to the feed solution;
   c. Introducing the feed solution into a spray-dryer and drying said GLP-1 peptide.
7. The process according to any one of the embodiments 1 to 4, wherein the pH of the feed solution is adjusted to pH 5-12.
8. The process according to any one of the preceding embodiments, wherein the pH of the feed solution is adjusted to pH 5-10.
9. The process according to any one of the preceding embodiments, wherein the pH of the feed solution is adjusted to pH 6-9.
10. The process according to any one of the preceding embodiments, wherein the pH of the feed solution is adjusted to pH 6-8.
11. The process according to any one of the preceding embodiments, wherein the feed solution pH is adjusted to the given a pH interval using a non-volatile base.
12. The process according to embodiment 11, wherein the non-volatile base is selected from alkali hydroxide such as sodium hydroxide or potassium hydroxide, a buffer such as a phosphate buffer, TRIS, acetate buffer.
13. The process according to any one of embodiments 11-12, wherein the non-volatile base is sodium hydroxide, e.g. 1M aqueous sodium hydroxide.
14. The process according to any one of the preceding embodiments, wherein the GLP-1 peptide is selected from semaglutide or Nε27-[2-[2-[2-[[2-[2-[2-[[(4S)-4-carboxy-4-[10-(4-carboxyphenoxy)decanoylamino]butanoyl]amino]ethoxy]ethoxy] acetyl]amino]ethoxy]ethoxy]acetyl], Nε36-[2-[2-[2-[[2-[2-[2-[[(4S)-4-carboxy-4-[10-(4-carboxyphenoxy)decanoylamino]butanoyl]amino]ethoxy]ethoxy]acetyl]amino]ethoxy]e thoxy]acetyl]-[Aib8,Glu22,Arg26,Lys27,Glu30,Arg34,Lys36]-GLP-1-(7-37)-peptidyl-Glu-Gly (Compound A).
15. The process according to any one of the preceding embodiments, wherein the feed solution solvent comprises a water miscible organic solvent.
16. The process according to any one of the preceding embodiments, wherein the feed solution solvent comprises an organic alcoholic solvent.
17. The process according to any one of the preceding embodiments, wherein the feed solution solvent comprises ethanol.
18. The process according to any one of the preceding embodiments, wherein the feed solution solvent is aqueous ethanol.
19. The process according to any one of the preceding embodiments, wherein the feed solution solvent is 40-70 percent (w/w) aqueous ethanol.
20. The process according to any one of the preceding embodiments, wherein the feed solution solvent is 45-65 percent (w/w) aqueous ethanol.
21. The process according to any one of the preceding embodiments, wherein the feed solution solvent is 50-60 percent (w/w) aqueous ethanol.
22. The process according to any one of the preceding embodiments, wherein the feed solution comprises 0.5-10 percent (w/w) of the GLP-1 peptide.
23. The process according to any one of the preceding embodiments, wherein the feed solution comprises 1-7 percent (w/w) of the GLP-1 peptide.
24. The process according to any one of the preceding embodiments, wherein the feed solution comprises 1.8-5 percent (w/w) of the GLP-1 peptide.
25. The process according to any one of the preceding embodiments, wherein the feed solution comprises 1-3 percent (w/w) of semaglutide.
26. The process according to any one of the preceding embodiments, wherein the feed solution comprises 1.8-2.1 percent (w/w) of semaglutide.
27. The process according to any one of the preceding embodiments, wherein the feed solution comprises 1-3 percent (w/w) semaglutide in 50-55 percent (w/w) ethanol.
28. The process according to any one of the preceding embodiments, wherein the feed solution comprises 1.8-2.1 percent (w/w) semaglutide in 50-55 percent (w/w) ethanol.
29. The process according to any one of embodiments 1-24, wherein the feed solution comprises 3-5 percent (w/w) of Compound A.
30. The process according to any one of embodiments 1-24 or 29, wherein the feed solution comprises 3-5 percent (w/w) Compound A in 50-65 percent (w/w) aqueous ethanol.
31. The process according to any one of the preceding embodiments, wherein the tablet produced comprises a therapeutically effective amount of the GLP-1 peptide.
32. The process according to any one of the preceding embodiments, wherein the tablet produced comprises the GLP-1 peptide in an amount of 0.01-100 mg.
33. The process according to any one of the preceding embodiments, wherein the tablet produced comprises the GLP-1 peptide in an amount of 0.1-50 mg.
34. The process according to any one of the preceding embodiments, wherein the tablet produced comprises at least one pharmaceutically acceptable excipient.
35. The process according to any one of the preceding embodiments, wherein the tablet produced comprises one or more excipients selected from the group consisting of binders, fillers, disintegrants, lubricants, delivery agents and/or glidants.
36. The process according to embodiment 35, wherein the lubricants is magnesium stearate.
37. The process according to any one of the preceding embodiments, wherein the tablet produced comprises a delivery agent.
38. The process according to embodiment 37, wherein the delivery agent is a salt of N-(8-(2-hydroxybenzoyl)amino)caprylic acid.
39. The process according to any one of embodiments 37-38, wherein the delivery agent is sodium N-(8-(2-hydroxybenzoyl)amino)caprylate.
40. The process according to any one of embodiments 37-39, wherein the delivery agent is present in an amount of at least 0.05 mmol.
41. The process according to any one of embodiments 37-40, wherein the delivery agent is present in an amount of 0.05-3 mmol.
42. The process according to any one of the preceding embodiments, wherein the tablet produced has a weight in the range of 150-1000 mg.
43. The process according to any one of the preceding embodiments, wherein the tablet produced has a weight in the range of 200-800 mg.
44. The process according to any one of the preceding embodiments, wherein the tablet produced has a weight in the range of 300-700 mg, such as 400 mg, 500 mg, 600 mg.
45. The process according to any one of the preceding embodiments, wherein the tablet produced is for oral administration.
46. A tablet obtained by the process according to any one of the preceding embodiments.
47. The tablet according to embodiment 46, wherein an improved intrinsic dissolution rate is obtained, e.g. as measured by the methods of Examples 1 and 2 herein.
48. The tablet according to any one of embodiments 46-47, wherein the tablet comprises a delivery agent.
49. The tablet according to any one of embodiments 48, wherein the delivery agents is sodium N-(8-(2-hydroxybenzoyl)amino)caprylate.
50. The tablet according to any one of embodiments 46-49 for use in medicine.
51. The tablet according to any one of embodiments 46-50 for the treatment of type 2 diabetes or obesity.
52. A method of treatment of type 2 diabetes comprising administering the tablet according to any one of embodiments 46-49 to a subject.
53. A method of treatment of obesity comprising administering the tablet according to any one of embodiments 46-49 to a subject.
54. Use of the tablet according to any one of embodiments 46-49 in the manufacture of a medicament for the treatment of type 2 diabetes.
55. Use of the tablet according to any one of embodiments 46-49 in the manufacture of a medicament for the treatment of obesity.

### EXAMPLES

### Abbreviations

MCC: Microcrystalline cellulose
MgSt: Magnesium stearate
PVP: Povidone K 90

### General Methods

### General method for spray-drying:

A feed solution of GLP-1 peptide in aqueous ethanol solvent was used for all experiments. Details on concentrations are given for each example. The pH of the feed solution was adjusted to given pH values using 1M NaOH or 1M HCl.

Spray-drying was carried out on a Büchi B-290 laboratory scale spray-dryer coupled with an inert loop B-295 for re-circulation of the drying gas optionally also coupled to a dehumidifier B-296. Nitrogen was used as drying gas and as atomizing gas. A two-fluid nozzle having a 0.7 mm inside diameter and cap 1.5 mm in diameter was used for atomising.

Condenser temperature set point was 0 °C or lower for all experiments and solvent used for stabilisation was aqueous ethanol with a concentration corresponding to the ethanol concentration of the feed solution.

The aspirator was switched on at 100%, followed by the nitrogen gas, at a level of 23-30 mm Hg on the built-in rotameter, corresponding to 0.35-0.50 kg/h of atomising gas. When sufficiently low oxygen levels were reached (<6 vol%) inside the spray-dryer, the heater was switched on at a set point of around 90 °C to heat up the spray-dryer (gas stabilisation). When the intended outlet temperature was reached (70-73 °C), the inlet temperature was increased to 110-136 °C. Once the target feed solution flow rate was reached (0.32-0.41 kg/h), the target outlet temperature was acquired by fine tuning the inlet temperature. All parameters were verified as required.

Once a stable profile was reached, the feeding tube of the spray-dryer was transferred from the stabilisation solvent to the actual feed solution comprising GLP-1 peptide as described above, to initiate the actual spray-drying process. Product was collected by a high-performance cyclone connected to the drying chamber for 2-56 minutes.

### Compact preparation

Compacts (also referred to herein as compact tablets) were made by compressing powder of GLP-1 peptide obtained from the General method for spray-drying described into compact tablets. More specifically, spray-dried GLP-1 peptide composition (20 to 50 mg) was compressed into a compact in a die (4 to 7 mm) using a pressure (0.5-1 metric ton) for 1 min.

### Tablet preparation

Tablets were prepared using two types of granules, SNAC/MCC/MgSt granules and MCC/PVP granules, as described in WO 2013/139695. SNAC/MCC/MgSt granules and MCC/PVP granules were prepared and mixed with extragranular semaglutide. The amount of MgSt included in Table 7 includes total amount and extra-granular material in parenthesis. Likewise, the total amount of MCC is included with the amounts of MCC in SNAC/MCC/MgSt and MCC/PVP granules, respectively, in parenthesis (Table 7).

A blend of 9.4 g of MCC/PVP granules and 0.6 g of MgSt was prepared using a 100 mL container and blending for 30 min at 25 rpm with a Pharmatech MB mixer. SNAC/MCC/MgSt granules for one tablet was weighed onto a weighing pan. On top of that, the blend of MCC/PVP granules and MgSt for one tablet was weighed. Semaglutide for one tablet was then weighed on top of the other components in the weighing pan. The components were then manually mixed and transferred directly to the tableting die cavity of the instrumented rotary tableting machine (Fette 102i). The mixture in the die cavity was compressed into an oval convex tablet with dimensions of 7.5 x 13 mm at a die table rotation speed of 20 rpm and a compression force around 9.5 kN.

### General method for intrinsic dissolution experiments (compact tablets) and dissolution experiments (tablets):

### Intrinsic dissolution experiments (compact tablets)

Intrinsic dissolution was used to analyse the variation on powder properties after spray-drying. A rotating disk apparatus (Woods apparatus) was used. The compact tablet was fitted in a tablet holder with one face exposed to the dissolution medium, the solid/liquid surface thereby remaining constant over the span of the analysis. The compact tablet was then rotated at a constant speed, and samples for concentration measurements withdrawn from the dissolution medium at pre-defined intervals. The amount of GLP-1 peptide dissolved in each sample was quantified by standard UPLC using UV detection at 215 nm or UV spectrometry using a Shimadzu UV spectrometer (215 nm). Intrinsic dissolution was measured according to pharmacopeia guidance (e.g. Ph. Eur 2.9.29; USP <1087>).

### Dissolution experiments (tablets)

The dissolution test was conducted with apparatus 2 in accordance with United States Pharmacopoeia 35 using a paddle rotation speed of 50 rpm. A 500 mL dissolution medium of 0.05 M phosphate buffer (pH 6.8) was used at a temperature of 37 °C. All dissolution media had a content of 0.05 % polyoxyethylene lauryl ether. Sample aliquots were removed at appropriate intervals for 1 hour and sample contents were determined using a RP-UHPLC method for dual detection of SNAC and GLP-1. The UHPLC method was based on gradient elution on a C18 column. The eluent system was 0.09 M (NH₄)₂HPO₄ (pH 3.6), acetonitrile and 2-propanol in water with UV detection at 210 and 335 nm. The sample contents were calculated based on the main peak area of the SNAC and semaglutide in the chromatogram relative to the main peak areas of the SNAC and semaglutide references, respectively. The released amounts of SNAC and semaglutide were calculated as percentages of the actual contents in the tablets determined by extracting all SNAC and semaglutide at 250 rpm for 10 min after the 1 hour of dissolution testing.

### Example 1: Intrinsic dissolution of semaglutide at various pH values.

A feed solution comprising 2 percent (w/w) semaglutide in 53 percent (w/w) aqueous ethanol was made as described under General Methods for spray-drying. From this stock solution, feed solutions of varying pH were made by adjusting pH using 1M HCl or 1M NaOH to obtain a pH range of 3-10 as measured using pH strips at room temperature and shown in Table 1. Spray-drying was performed using a Büchi B-290 spray-dryer with parameter: Feed flow rate: 0.38-0.41 kg/h; Atomising gas flow rate: 0.50 kg/h; Outlet temperature: 73 °C; Inlet temperature: 110-114 °C.

**Table 1: Feed solutions used for spray-drying of semaglutide.**

| **Exp. no.** | **pH** | **EtOH conc** (w/w%) | **Semaglutide conc** (w/w%) |
|---|---|---|---|
| 1 | 3 | 53 | 2 |
| 2 | 3.5 | 53 | 2 |
| 3 | 5 | 53 | 2 |
| 4 | 5 | 53 | 2 |
| 5 | 7 | 53 | 2 |
| 6 | 10 | 53 | 2 |

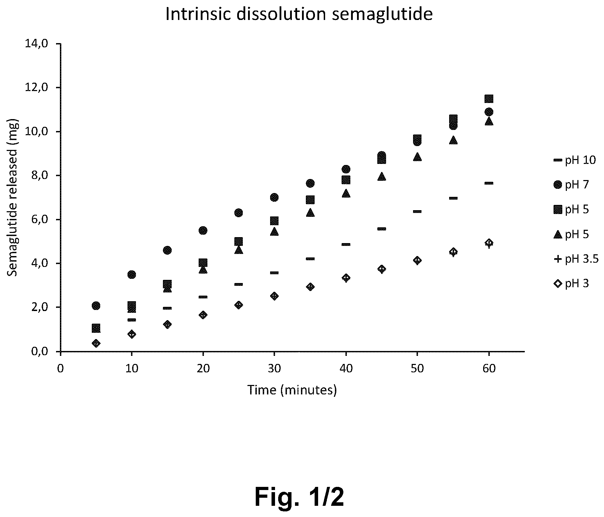

From each of experiment numbers 1-6, 20 mg of the obtained spray-dried powder of semaglutide was compressed into a compact tablet in a 4 mm die using a pressure of 0.5 metric ton for 1 min. Each compact tablet was tested for intrinsic dissolution using the general method for intrinsic dissolution testing described above. Dissolution medium was 500 ml of a 50 mM phosphate buffer at pH 6.8 containing 0.2 percent Brij 35^{®}. Test conditions used were a temperature of 25 °C at a rotational speed of 75 rpm. Samples of 1 ml were drawn from each dissolution vessel every 5 minutes and subsequently the content of dissolved semaglutide in the sample was quantified using UPLC analysis. The results are presented in Table 2 and Figure 1.

**Table 2: Intrinsic dissolution of tablets comprising semaglutide, wherein semaglutide was obtained by spray-drying at various pH values. Each experiment is an average of 2-3 individual compact tablets.**

| **Ex no** | **pH** | **Dissolved compound (mg) at given time (min)** | | | | | | | | | | | |
|---|---|---|---|---|---|---|---|---|---|---|---|---|---|
| | | **5** | **10** | **15** | **20** | **25** | **30** | **35** | **40** | **45** | **50** | **55** | **60** |
| 1 | 3 | 0.38 | 0.79 | 1.23 | 1.66 | 2.09 | 2.50 | 2.93 | 3.34 | 3.74 | 4.14 | 4.53 | 4.94 |
| 2 | 3.5 | 0.39 | 0.83 | 1.25 | 1.67 | 2.07 | 2.49 | 2.91 | 3.29 | 3.69 | 4.09 | 4.45 | 4.84 |
| 3 | 5 | 1.05 | 1.96 | 2.87 | 3.73 | 4.62 | 5.46 | 6.32 | 7.18 | 7.95 | 8.86 | 9.62 | 10.5 |
| 4 | 5 | 1.05 | 2.06 | 3.05 | 4.02 | 4.99 | 5.94 | 6.88 | 7.79 | 8.72 | 9.66 | 10.6 | 11.5 |
| 5 | 7 | 2.06 | 3.48 | 4.59 | 5.50 | 6.30 | 6.99 | 7.64 | 8.28 | 8.91 | 9.53 | 10.3 | 10.9 |
| 6 | 10 | 1.12 | 1.43 | 1.94 | 2.45 | 3.03 | 3.56 | 4.21 | 4.86 | 5.57 | 6.36 | 6.95 | 7.64 |

The intrinsic dissolution rates for semaglutide are shown in Table 3 and were calculated based on the data in Table 2. The intrinsic dissolution rate was calculated using the slope from linear regression of the released amount of semaglutide of the linear part of the intrinsic dissolutions and the release area of 0.1257 cm². The linear regressions were based on all time points for exp. 1-4 and 6 whereas the first three time points at 5, 10, and 15 min of the intrinsic dissolution were excluded for exp. 5 due to non-linearity.

**Table 3: Intrinsic dissolution rates of compacts comprising semaglutide at 25 °C.**

| **Experiment no.** | **pH** | **Intrinsic dissolution rate** (mg/(min*cm²) |
|---|---|---|
| 1 | 3 | 0.7 |
| 2 | 3.5 | 0.6 |
| 3 | 5 | 1.4 |
| 4 | 5 | 1.5 |
| 5 | 7 | 1.1 |
| 6 | 10 | 1.0 |

The data in Table 3 and Figure 1 shows that when semaglutide obtained by spray-drying from a feed solution with a pH above 3.5 results in higher intrinsic dissolution rates of semaglutide (exp. 3-6) than when the pH of the feed solution is 3.5 or lower (exp. 1-2). Furthermore, the intrinsic dissolution rates in Table 1 show that the intrinsic dissolution rate for semaglutide is the highest when the pH of the feed solution is around 5.

### Example 2: Intrinsic dissolution of Compound A at various pH values.

A feed solution of 30-40 mg/ml Nε27-[2-[2-[2-[[2-[2-[2-[[(4S)-4-carboxy-4-[10-(4-carboxyphenoxy)decanoylamino]butanoyl]amino]ethoxy]ethoxy] acetyl]amino]ethoxy]ethoxy]acetyl], Nε36-[2-[2-[2-[[2-[2-[2-[[(4S)-4-carboxy-4-[10-(4-carboxyphenoxy)decanoylamino]butanoyl]amino]ethoxy]ethoxy]acetyl]amino]ethoxy]ethoxy]a cetyl]-[Aib8,Glu22,Arg26,Lys27,Glu30,Arg34,Lys36]-GLP-1-(7-37)-peptidyl-Glu-Gly (Compound A) in 50-63 percent (w/w) aqueous ethanol was prepared as described under General Methods and pH was measured to 5 using pH strips at room temperature. From this stock solution, feed solutions of varying pH were made by adjusting pH using 1M HCI or 1M NaOH to obtain a pH range of 3.6-7 as measured with pH strips at room temperature. Optionally, NaCl was added at a concentration of 5 mg/ml according to Table 4. Spray-drying parameters used on Büchi B-290: Feed flow rate: 6 ml/min (corresponding to 0.32-0.33 kg/h); Atomising gas flow rate: 0.35-0.50 kg/h; Outlet temperature: 70 °C; Inlet temperature: 120-136 °C.

**Table 4: Feed solutions used for spray-drying of Compound A. * comprises NaCl; ^{#} had larger particles, ^{##} had smaller particles.**

| **Exp. no.** | **pH** | **EtOH conc** (w/w%) | **Compound A conc** (mg/ml) | **NaCl conc** (mg/ml) |
|---|---|---|---|---|
| 7 | 3.6 | 50 | 30 | 0 |
| 8 | 5^{#} | 63 | 40 | 0 |
| 9 | 7 | 50 | 30 | 0 |
| 10 | 7* | 63 | 30 | 5 |
| 11 | 5^{##} | 70 | 22 | 0 |

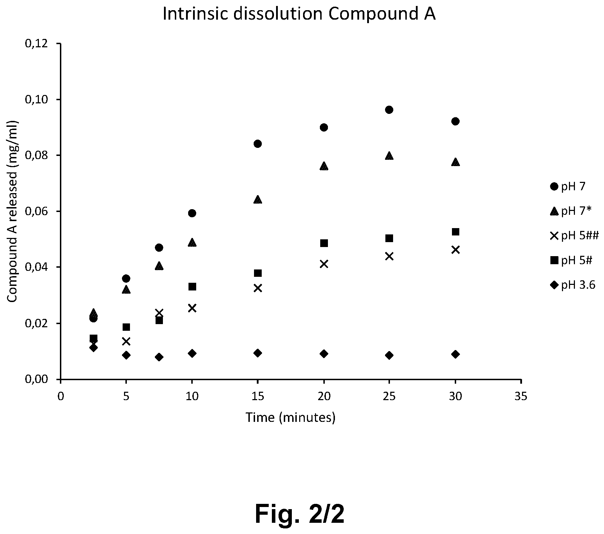

From each of experiment numbers 7-10, 50 mg of the obtained spray-dried powder of Compound A was compressed into a compact tablet in a 7 mm die using a pressure of 1 metric ton for 1 min. Each compact tablet was tested for intrinsic dissolution using the general method for intrinsic dissolution testing. Dissolution medium was 500 ml of a 50 mM sodium diphosphate buffer at pH 6.8 containing 0.05 percent Brij 35^{®}. Test conditions used were a temperature of 30 °C at a rotational speed of 50 rpm. Samples of 1 ml were drawn from each dissolution vessel every 2.5 min for 10 min followed by every 5 min for additional 20 min and subsequently the content of dissolved Compound A in the sample was quantified by UV spectrometry (215 nm). The results are presented in Table 5 and Figure 2. For comparison, a compact tablet was also made directly from the initial powder of Compound A used to make the stock solution. Said powder was obtained by a different spray-drying method resulting in smaller particles which were included in the intrinsic dissolution experiment to evaluate if the dissolution was dependent on particle size. The feed solution for spray-drying had a pH of 5 (Exp. no. 11).

The results are presented in Table 5 and Figure 2.

**Table 5: Intrinsic dissolution of tablets comprising Compound A, wherein Compound A was obtained at various pH values. * comprises NaCl; ^{#} had larger particles, ^{##} had smaller particles.**

| **Exp. no.** | **pH** | **Dissolved compound (mg/ml) at given time (min)** | | | | | | | |
|---|---|---|---|---|---|---|---|---|---|
| | | **2.5** | **5** | **7.5** | **10** | **15** | **20** | **25** | **30** |
| 7 | 3.6 | 0.011 | 0.009 | 0.008 | 0.009 | 0.009 | 0.009 | 0.009 | 0.009 |
| 8 | 5^{#} | 0.015 | 0.019 | 0.021 | 0.033 | 0.038 | 0.049 | 0.050 | 0.053 |
| 9 | 7 | 0.022 | 0.036 | 0.047 | 0.059 | 0.084 | 0.090 | 0.096 | 0.092 |
| 10 | 7* | 0.024 | 0.032 | 0.041 | 0.049 | 0.064 | 0.076 | 0.080 | 0.078 |
| 11 | 5^{##} | 0.013 | 0.014 | 0.024 | 0.025 | 0.033 | 0.041 | 0.044 | 0.046 |

The intrinsic dissolution rates for compound A are shown in Table 6 and were calculated based on the data in Table 5. The intrinsic dissolution rate was calculated using the slope from linear regression of the released amount of compound A of the linear part of the intrinsic dissolutions and the release area of 0.3848 cm². The linear regressions were based on all time points for exp. 7 whereas the last two time points at 25 and 30 min and the last three time points at 20, 25, and 30 min of the intrinsic dissolutions were excluded for exp. 8 and 11 and for exp. 9-10, respectively, due to non-linearity.

**Table 6: Intrinsic dissolution rates of compacts comprising Compound A at 30 °C.**

| **Experiment no.** | **pH** | **Intrinsic dissolution rate (mg/(min*cm²)** |
|---|---|---|
| 7 | 3.6 | 0.0 |
| 8 | 5 | 2.6 |
| 9 | 7 | 6.4 |
| 10 | 7 | 4.2 |
| 11 | 5 | 2.1 |

The data in Table 6 and Figure 2 show that spray-dried Compound A obtained by a process according to the invention, i.e. spray-drying a feed solution comprising the GLP-1 peptide and a feed solution solvent, wherein the pH of the feed solution is in the range of about 5 to about 10 (Exp. No. 8-11), had a higher intrinsic dissolution rate compared to a spray-dried Compound A obtained by spray-drying a feed solution comprising the GLP-1 peptide and a feed solution solvent, wherein the pH of the feed solution is in the range of below 5 (Exp. No 7).

### Example 3: Dissolution testing of tablets comprising spray-dried semaglutide.

Different samples of spray-dried semaglutide powder were manufactured by spray-drying as described in Example 1. The so-obtained spray-dried semaglutide powder was compressed into tablets following the procedure described in "Tablet preparation". The tablet compositions are shown in Table 7. Each experiment is an average of 6 individual tablets.

**Table 7: Tablet compositions comprising spray-dried semaglutide (mg/tablet).**

| **Tablet** | **Semaglutide** | | | | | **SNAC** | **MCC** | **PVP** | **MgSt** |
|---|---|---|---|---|---|---|---|---|---|
| | **pH 2.7** | **pH 3.5** | **pH 5.0** | **pH 7.1** | **pH 10.3** | | | | |
| I | 3 | - | - | - | - | 300 | 80 (57/23) | 8 | 9.7 (2) |
| II | - | 3 | - | - | - | 300 | 80 (57/23) | 8 | 9.7 (2) |
| III | - | - | 3 | - | - | 300 | 80 (57/23) | 8 | 9.7 (2) |
| IV | - | - | - | 3 | - | 300 | 80 (57/23) | 8 | 9.7 (2) |
| V | - | - | - | - | 3 | 300 | 80 (57/23) | 8 | 9.7 (2) |

Dissolution tests were conducted on Tablets I to V as described under "Dissolution experiments". The results are shown in Table 8.

**Table 8: Dissolution of semaglutide formulated into a tablet.**

| **Tablet** | **Dissolved semaglutide at given time (% of total semaglutide in tablet)** | | | | |
|---|---|---|---|---|---|
| | **0 min** | **15 min** | **30 min** | **45 min** | **60 min** |
| I | 0 | 44 | 67 | 80 | 86 |
| II | 0 | 44 | 70 | 82 | 89 |
| III | 0 | 45 | 70 | 84 | 91 |
| IV | 0 | 45 | 71 | 85 | 91 |
| V | 0 | 46 | 72 | 85 | 91 |

The dissolution results for semaglutide (Tablets I to V, Table 8) show that the lower the pH during the spray-drying of semaglutide the slower the dissolution and release of semaglutide from the tablets. Specifically, the results in Table 8 show that when the semaglutide of the tablet is obtained by spray-drying from a feed solution with a pH of 5 or higher (Tablets III-V), then a higher dissolution rate of the resulting tablet is obtained compared to a pH below 5 (Tablets I-II). Since Tablets I-V are so-called immediate release tablets with normal dissolution then it is the time points of 30 and 45 minutes for Tablets I-V which are appropriate to use for a comparison between these tablets of the dissolution results (this is in line with e.g. section 2.4.1 in <1092> "The dissolution procedure: Development and validation" of the US Pharmacopoeia which states that immediate release tablets typically reach 85-100% release within 30-45 minutes; the "Reflection paper on the dissolution specification for generic solid oral immediate release products with systemic action" published by the European Medicines Agency (EMA/CHMP/CVMP/QWP/336031/2017) stating that an immediate release tablets should be able to release ≥ 75% within 45 min; and ICH guideline Q6A (decision tree #7-1) which defines "normal dissolution" for immediate release tablets as a dissolution of >80% after 15 minutes as opposed to "rapid dissolution" as >80% within 15 minutes for immediate release tablets). As can be seen in Table 8, the amount of dissolved semaglutide at the time points 30 min and 45 min is higher for Tablets III-V than for Tablets I and II.

## Claims

1. A process of producing a tablet comprising a GLP-1 peptide, said method comprising the steps of:
a. Spray-drying a feed solution comprising the GLP-1 peptide and a feed solution solvent, wherein the pH of the feed solution is in the range of about 5 to about 10, such as in the range of 5-10;
b. Compressing the obtained powder of GLP-1 peptide and optionally at least one pharmaceutically acceptable excipient into a tablet.

2. The process according to claim 1 comprising the steps of:
a. Dissolving the GLP-1 peptide in the feed solution solvent or obtaining the GLP-1 peptide in solution directly from a purification step;
b. Adjusting pH of the feed solution to a pH of about 5 to about 10, such as in the range of 5-10, by addition of a non-volatile base to the feed solution;
c. Introducing the feed solution into a spray-dryer and drying said GLP-1 peptide;
d. Compressing the obtained powder of GLP-1 peptide and optionally at least one pharmaceutically acceptable excipient into a tablet.

3. The process according to any one of the preceding claims, wherein the feed solution pH is adjusted to a pH higher than pl of the GLP-1 peptide or the range of pH 5-10 using a non-volatile base.

4. The process according to claim 3, wherein the non-volatile base is selected from alkali hydroxide such as sodium hydroxide or potassium hydroxide, e.g. 1 M aqueous sodium hydroxide; a buffer such as a phosphate buffer, TRIS, acetate buffer.

5. The process according to any one of the preceding claims, wherein the GLP-1 peptide is semaglutide.

6. The process according to any one of claims 1-4, wherein the GLP-1 peptide is Nε27-[2-[2-[2-[[2-[2-[2-[[(4S)-4-carboxy-4-[10-(4-carboxyphenoxy)decanoylamino]butanoyl]amino]ethoxy]ethoxy] acetyl]amino]ethoxy]ethoxy]acetyl], Nε36-[2-[2-[2-[[2-[2-[2-[[(4S)-4-carboxy-4-[10-(4-carboxyphenoxy)decanoylamino]butanoyl]amino]ethoxy]ethoxy]acetyl]amino]ethoxy]e thoxy]acetyl]-[Aib8,Glu22,Arg26,Lys27,Glu30,Arg34,Lys36]-GLP-1-(7-37)-peptidyl-Glu-Gly (Compound A).

7. The process according to claim 5 wherein the pH of the feed solution comprising semaglutide is in the range of about 6 to about 10 or about 6 to about 8 or such as about 8.5 to about 9.5.

8. The process according to claim 6, wherein the pH of the feed solution comprising Compound A is about 6 to about 10 or about 7 to about 9.

9. The process according to any one of the preceding claims, wherein the feed solution solvent is aqueous ethanol.

10. The process according to any one of the preceding claims, wherein the feed solution solvent is 40-70 percent (w/w) aqueous ethanol.

11. The process according to any one of the preceding claims, wherein the feed solution comprises 0.5-10 percent (w/w) of the GLP-1 peptide.

12. A tablet obtained by the process according to any one of the preceding claims.

13. The tablet according to claim 12, wherein the tablet comprises a delivery agent.

14. The tablet according to claim 13, wherein the delivery agent is sodium N-(8-(2-hydroxybenzoyl)amino)caprylate.

15. The tablet according to any of the claims 12-14, wherein the weight of the tablet is in the range of 100 mg to 1000 mg.

16. The tablet according to any of the claims 12-15, wherein the tablet comprises 0.2 to 80 mg GLP-1 peptide.

17. The tablet according to any one of claims 12-16 for use in medicine.

18. The tablet according to any one of claims 12-17 for use in the treatment of type II diabetes or obesity.

## Patentansprüche

1. Verfahren für ein Herstellen einer Tablette, umfassend ein GLP-1-Peptid, das Verfahren die folgenden Schritte umfassend:
a. Sprühtrocknung einer Beschickungslösung umfassend das GLP-1-Peptid und ein Beschickungslösungsmittel, wobei der pH-Wert der Beschickungslösung in einem Bereich von etwa 5 bis etwa 10, wie in einem Bereich von 5-10, liegt;
b. Komprimieren des erhaltenen Pulvers aus GLP-1-Peptid und gegebenenfalls mindestens eines pharmakologisch verträglichen Exzipienten zu einer Tablette.

2. Verfahren nach Anspruch 1, umfassend die folgenden Schritte:
a. Auflösen des GLP-1-Peptids in der Beschickungslösung oder Gewinnen des GLP-1-Peptids in Lösung direkt aus einem Reinigungsschritt;
b. Einstellen des pH-Wertes der Beschickungslösung auf einen pH-Wert von etwa 5 bis etwa 10, beispielsweise in einem Bereich von 5-10, durch Zugabe einer nichtflüchtigen Base zu der Beschickungslösung;
c. Einbringen der Beschickungslösung in einen Sprühtrockner und Trocknen des GLP-1-Peptids;
d. Komprimieren des erhaltenen Pulvers aus GLP-1-Peptid und gegebenenfalls mindestens eines pharmakologisch verträglichen Exzipienten zu einer Tablette.

3. Verfahren nach einem der vorhergehenden Ansprüche, wobei der pH-Wert der Beschickungslösung unter Verwendung einer nichtflüchtigen Base auf einen pH-Wert höher als pl des GLP-1-Peptids oder den Bereich von pH 5-10 eingestellt wird.

4. Verfahren nach Anspruch 3, wobei die nichtflüchtige Base ausgewählt ist aus Alkalihydroxid wie Natriumhydroxid oder Kaliumhydroxid, z. B. 1M wässriges Natriumhydroxid; einem Puffer wie einem Phosphatpuffer, TRIS, Acetatpuffer.

5. Verfahren nach einem der vorhergehenden Ansprüche, wobei das GLP-1-Peptid Semaglutid ist.

6. Verfahren nach einem der Ansprüche 1-4, wobei das GLP-1-Peptid Nε27-[2-[2-[2-[[2-[2-[2-[[(4S)-4-Carboxy-4-[10-(4-carboxyphenoxy)decanoylamino]butanoyl]amino]ethoxy]ethoxy]ethoxy]acetyl]amino]etho xy]ethoxy]acetyl], Nε36-[2-[2-[2-[[2-[2-[2-[[(4S)-4-Carboxy-4-[10-(4-carboxyphenoxy)decanoylamino]butanoyl]amino]ethoxy]ethoxy]acetyl]amino]ethoxy]etho xy]acetyl]-[Aib8,Glu22,Arg26,Lys27,Glu30,Arg34,Lys36]-GLP-1-(7-37)-peptidyl-Glu-Gly (Verbindung A) ist.

7. Verfahren nach Anspruch 5, wobei der pH-Wert der Semaglutid umfassenden Beschickungslösung in dem Bereich von etwa 6 bis etwa 10 oder etwa 6 bis etwa 8 oder beispielsweise etwa 8,5 bis etwa 9,5 liegt.

8. Verfahren nach Anspruch 6, wobei der pH-Wert der die Verbindung A umfassenden Beschickungslösung etwa 6 bis etwa 10 oder etwa 7 bis etwa 9 beträgt.

9. Verfahren nach einem der vorhergehenden Ansprüche, wobei das Lösungsmittel der Beschickungslösung wässriges Ethanol ist.

10. Verfahren nach einem der vorhergehenden Ansprüche, wobei das Lösungsmittel der Beschickungslösung 40-70 % (w/w) wässriges Ethanol ist.

11. Verfahren nach einem der vorhergehenden Ansprüche, wobei die Beschickungslösung 0,5-10 Prozent (w/w) des GLP-1-Peptids umfasst.

12. Tablette, erhalten durch das Verfahren nach einem der vorhergehenden Ansprüche.

13. Tablette nach Anspruch 12, wobei die Tablette ein Abgabemittel umfasst.

14. Tablette nach Anspruch 13, wobei das Abgabemittel Natrium-N-(8-(2-hydroxybenzoyl)amino)caprylat ist.

15. Tablette nach einem der Ansprüche 12-14, wobei das Gewicht der Tablette in dem Bereich von 100 mg bis 1000 mg liegt.

16. Tablette nach einem der Ansprüche 12-15, wobei die Tablette 0,2 bis 80 mg GLP-1-Peptid umfasst.

17. Tablette nach einem der Ansprüche 12-16 für eine Verwendung in der Medizin.

18. Tablette nach einem der Ansprüche 12-17 für eine Verwendung bei der Behandlung von Typ-II-Diabetes oder Adipositas.

## Revendications

1. Procédé de production d'un comprimé comprenant un peptide GLP-1, ledit procédé comprenant les étapes de :
a. le séchage par pulvérisation d'une solution d'alimentation comprenant le peptide GLP-1 et un solvant de solution d'alimentation, dans lequel le pH de la solution d'alimentation est dans la plage d'environ 5 à environ 10, par exemple dans la plage de 5 à 10 ;
b. la compression de la poudre obtenue de peptide GLP-1 et éventuellement de l'au moins un excipient pharmaceutiquement acceptable en un comprimé.

2. Procédé selon la revendication 1, comprenant l'étape consistant à :
a. dissoudre le peptide GLP-1 dans le solvant de la solution d'alimentation ou l'obtention du peptide GLP-1 en solution directement à partir d'une étape de purification ;
b. ajuster le pH de la solution d'alimentation à un pH d'environ 5 à environ 10, par exemple dans la plage de 5 à 10, par l'ajout d'une base non volatile à la solution d'alimentation ;
c. introduire la solution d'alimentation dans un sécheur par pulvérisation et le séchage dudit peptide GLP-1 ;
d. comprimer la poudre obtenue de peptide GLP-1 et éventuellement de l'au moins un excipient pharmaceutiquement acceptable en un comprimé.

3. Procédé selon l'une quelconque des revendications précédentes, dans lequel le pH de la solution d'alimentation est ajusté à un pH supérieur à pl du peptide GLP-1 ou dans la plage de pH 5-10 en utilisant une base non volatile.

4. Procédé selon la revendication 3, dans lequel la base non volatile est choisie parmi l'hydroxyde alcalin tel que l'hydroxyde de sodium ou l'hydroxyde de potassium, par exemple l'hydroxyde de sodium aqueux 1 M ; un tampon tel qu'un tampon phosphate, un tampon TRIS, un tampon acétate.

5. Procédé selon l'une quelconque des revendications précédentes, dans lequel le peptide GLP-1 est sémaglutide.

6. Procédé selon l'une quelconque des revendications 1 à 4, dans laquelle le peptide GLP-1 est Nε27-[2-[2-[2-[[2-[2-[2-[[(4S)-4-carboxy-4-[10-(4-carboxyphenoxy)décanoylamino]butanoyl]amino]ethoxy]ethoxy] acétyl]amino]éthoxy]éthoxy]acétyl], Nε36-[2-[2-[2-[[2-[2-[2-[[(4S)-4-carboxy-4-[10-(4-carboxyphénoxy)décanoylamino]butanoyl]amino]ethoxy]éthoxy]acétyl]amino]éthoxy]étho xy]acétyl]-[Aib8,Glu22,Arg26,Lys27,Glu30,Arg34,Lys36]-GLP-1-(7-37)-peptidyl-Glu-Gly (Composé A).

7. Procédé selon la revendication 5, dans lequel le pH de la solution d'alimentation comprenant le sémaglutide est dans la plage d'environ 6 à environ 10 ou d'environ 6 à environ 8 ou tel qu'environ 8,5 à environ 9,5.

8. Procédé selon la revendication 6, dans lequel le pH de la solution d'alimentation comprenant le Composé A est d'environ 6 à environ 10 ou d'environ 7 à environ 9.

9. Procédé selon l'une quelconque des revendications précédentes, dans lequel le solvant de la solution d'alimentation est l'éthanol aqueux.

10. Procédé selon l'une quelconque des revendications précédentes, dans lequel le solvant de la solution d'alimentation est à 40 à 70 pour cent (p/p) l'éthanol aqueux.

11. Procédé selon l'une quelconque des revendications précédentes, dans lequel la solution d'alimentation comprend 0,5 à 10 pour cent (p/p) du peptide GLP-1.

12. Comprimé obtenu par le procédé selon l'une quelconque des revendications précédentes.

13. Comprimé selon la revendication 12, dans lequel le comprimé comprend un agent de libération.

14. Comprimé selon la revendication 13, dans lequel l'agent de libération est le N-(8-(2-hydroxybenzoyl)amino)caprylate de sodium.

15. Comprimé selon l'une quelconque des revendications 12 à 14, dans lequel ledit poids du comprimé est dans la plage de 100 à 1 000 mg.

16. Comprimé selon l'une quelconque des revendications 12 à 15, dans lequel le comprimé comprend 0,2 à 80 mg de peptide GLP-1.

17. Comprimé selon l'une quelconque des revendications 12 à 16 pour une utilisation en médecine.

18. Comprimé selon l'une quelconque des revendications 12 à 17, pour utilisation dans le traitement du diabète de type II ou de l'obésité.
